(19) Europäisches Patentamt
European Patent Office
Office européen des brevets

(11) Veröffentlichungsnummer: **0 010 056**
**B1**

(12) # EUROPÄISCHE PATENTSCHRIFT

(45) Veröffentlichungstag der Patentschrift: **24.08.83**

(21) Anmeldenummer: **79710095.5**

(22) Anmeldetag: **03.10.79**

(51) Int. Cl.³: **C 07 J 31/00,**
C 07 J 41/00, C 07 J 51/00,
C 07 J 53/00, C 07 J 63/00,
C 07 J 71/00,
A 61 K 31/57,
A 61 K 31/585 //C12P33/08

(54) **Kortikoid-21-sulfopropionate und deren Salze, Verfahren zu ihrer Herstellung und pharmazeutische Präparate, die diese Verbindungen enthalten.**

(30) Priorität: **04.10.78 DE 2843690**
**06.08.79 DE 2932166**

(43) Veröffentlichungstag der Anmeldung:
**16.04.80 Patentblatt 80/8**

(45) Bekanntmachung des Hinweises auf die Patenterteilung:
**24.08.83 Patentblatt 83/34**

(84) Benannte Vertragsstaaten:
**AT BE CH DE FR GB IT LU NL SE**

(56) Entgegenhaltungen:
**DE - B - 1 090 207**
**FR - A - 1 392 048**
**US - A - 3 089 881**

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Laurent, Henry, Dr.**
**Glambecker Weg 21**
**D-1000 Berlin 28 (DE)**
Erfinder: **Esperling, Peter**
**Furkastrasse 15 C**
**D-1000 Berlin 42 (DE)**
Erfinder: **Kapp, Joachim-Friedrich, Dr.**
**Ludolfinger Weg 51**
**D-1000 Berlin 28 (DE)**
Erfinder: **Wiechert, Rudolf, Prof.**
**Petzower Strasse 8 A**
**D-1000 Berlin 39 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 010 056 B1

# Kortikoid-21-sulfopropionate und deren Salze, Verfahren zu ihrer Herstellung und pharmazeutische Präparate, die diese Verbindungen enthalten

Die Erfindung betrifft den in den Patentansprüchen gekennzeichneten Gegenstand.

Als Acyloxygruppen $R_1$ der Kortikoid-21-sulfopropionate der allgemeinen Formel I gemäß Anspruch 1 kommen vorzugsweise solche in Betracht, die sich von einer gesättigten, geradkettigen oder verzweigten, aliphatischen Carbonsäuren (wie zum Beispiel der Ameisensäure, Essigsäure, Propionsäure, Buttersäure, Isobuttersäure, Pivalinsäure, Valeriansäure oder Capronsäure/ oder der Benzoesäure ableiten.

Die neuen Kortikoid-21-sulfopropionate können in einfacher Weise hergestellt werden, indem man die entsprechenden 21-Hydroxysteroide in einem aprotischen Lösungsmittel mit einem Überschuß an 3-Sulfopropionsäureanhydrid umsetzt. Geeignete Lösungsmittel sind beispielsweise Kohlenwasserstoffe, wie Benzol oder Toluol, halogenierte Kohlenwasserstoffe, wie Methylenchlorid, Chloroform, Tetrachlorkohlenstoff, Äthylenchlorid oder Tetrachloräthan, Äther wie Diisopropyläther, Tetrahydrofuran, Dioxan oder Dimethoxyäthan oder Ketone, wie Aceton oder Methylisobutylketon.

Die Reaktion wird vorzugsweise bei einer Reaktionstemperatur von 0 bis 30°C durchgeführt. Bei der Reaktion werden vorzugsweise pro Mol Kortikoid 1,1 bis 10 Mol 3-Sulfopropionsäureanhydrid eingesetzt.

Die erhaltenen Kortikoid-21-sulfopropionate lassen sich gegebenenfalls mittels physiologisch unbedenklichen Basen in ihre Salze überführen. Geeignete Salze sind beispielsweise Alkalimetallsalze (vorzugsweise Lithium, Natrium oder Kaliumsalze), Erdalkalimetallsalze (vorzugsweise Magnesium- oder Calziumsalze), oder Salze von Amine. Geeignete Amine sind beispielsweise aliphatische, cycloaliphatische oder araliphatische Amine mit 1 bis 8 Kohlenstoffatomen und heterocyclische Aminie, z.B. Mono- Di- und Triäthylamin, Mono-, Di- und Trimethylamin, Mono-, Di- und Triisopropylamin, Äthyldimethylamin, Benzyldiäthylamin, Cyclohexylamin, Dibenzylamin, N,N-Dibenzyläthyldiamin, Bis-o-methoxy-phenylisopropylamin, Methoxyphenylisopropylamin, Piperidin, Morpholin, Pyrrolidin, Piperazin und niedere Alkylderivate davon, wie 1-Methylpiperridin, 4-Äthylmorpholin, 1-Isopropylpyrrolidin, 1,4-Dimethylpiperazin, 1-n-Butylpiperidin, 2-Methylpiperidin, 1-Methyl-2-methylpiperidin, ferner Amine mit wasserlöslichen oder hydrophilen Gruppen, wie Mono-, Di- und Triäthanolamin, Äthyldiäthanolamin, N-Butylmonoäthanolamin, 2-Amino-1-butanol, 2-Amino-2-äthyl-1,3-propandiol, 2-Amino-2-methyl-1-propanol, Phenylmonoäthanolamin, p-tert.-Amylphenyldiäthanolamin, Galactosamin, N-Methylglucamin, N-Methylglucosamin, Ephedrin, Phenylephrin, Epinephrin, Procain, 2-(4'-tert.-Butyl-2',6'-dimethyl-phenyl-methyl)-imidazolin.

Die neuen Kortikoid-21-sulfopropionate und insbesondere deren Salze mit physiologisch unbedenklichen Basen zeichnen sich gegenüber den freien 21-Hydroxykortikoiden dadurch aus, daß sie in Wasser gut lösliche Verbindungen sind.

Wasserlösliche Derivate von Kortikoiden sind bekannt und werden in der Therapie seit langem angewendet. Derartige Derivate sind beispielsweise Natriumsalze von Kortikoid-2-hemisulfaten, Kortikoid-21-phosphaten, Kortikoid-21-sulfobenzoaten, Kortikoid-21-hemisuccinaten und Kortikoid-21-aminoacylaten.

Die Natriumsalze von Kortikoid-21-hemisulfaten, Kortikoid-21-phosphaten und Kortikoid-21-sulfobenzoaten bilden in der Regel stabile, sterilisierbare und lagerbare Lösungen. Sie haben aber den Nachteil, daß sie nach i.v.-Applikation nur relativ langsam und meist sogar nur unvollständig in die freien Kortikoide (d.h. die eigentlichen Wirkstoffe) gespalten werden. Dieser Nachteil wirk sich besonders gravierend bei der Behandlung lebensgefährlicher Schockzustände mit derartigen Präparaten aus.

Die Natriumsalze von Kortikoid-21-hemisuccinaten und Kortikoid-21-aminoacylaten andererseits werden nach i.v.-Applikation sehr rasch gespalten, so daß die eigentlichen Kortikoidwirkstoffe sofort ihre volle Wirksamkeit entfalten können. Die Lösungen dieser Stoffe sind aber so instabil, baß sie weder heißsterilisiert noch bei Raumtemperatur gelagert werden können. Deshalb sind die diese Wirkstoffe enthaltenden Präparate stets Trockenpulver, die kurz vor der Applikation gelöst werden müssen. Dies ist ein nicht zu unterschätzender Nachteil, da die Gefahr recht groß ist, daß die so hergerstellten Injektionslösungen unsteril sind und daß ein Teil des Wirkstoffes ungelöst bleibt.

Demgegenüber haben die Salze der erfindungsgemäßen Kortikoid-21-sulfopropionate den Vorzug, daß ihre wässrige Lösungen so stabil sind, daß sie hitzesterilisiert und gelagert werden können und zudem nach der i.v.-Applikation sehr rasch gespalten werden, wie aus den nachfolgenden Ergebnissen des Endotoxin-Schock-Testes ersichtlich ist.

Zum Endotoxin-Schock-Test wurden jeweils 10 Ratten im Gewicht von 100—120 g adrenalektomiert und erhielten am kommenden Tage unter leichter Äthernarkose 5 $\mu$g Endotoxin pro 100 g Körpergewicht intravenös appliziert. Unmittelbar nach dieser Injektion wurde die Kortikoidlösung durch die gleiche Kanüle appliziert. Es wurde die Anzahl an Tieren bestimmt die diese Behandlung 24 Stunden lang überlebten.

Ergebnisse des Endotoxin-Schock-Testes

| Nr. | Substanz | Dosis in mg/kg Tier | Zahl der überlebenden Tiere |
|---|---|---|---|
| | A) Derivate des Dexamethasons (= 9α-Fluor-11β,17α,21-trihydroxy-16α-methyl-1,4-pregnadien-3,20-dion) | | |
| 1) | Dexamethason-21-hemisulfat-Natrium | 0,03<br>0,3 | 0<br>1 |
| 2) | Dexamethason-21-o-sulfobenzoat-Natrium | 0,03<br>0,3 | 2<br>8 |
| 3) | Dexamethason-21-(3-sulfopropionat)-Natrium | 0,03<br>0,3 | 10<br>10 |
| | B) Derivate des Methylprednisolons (=11β,17α,21-Trihydroxy-6α-methyl-1,4-pregnadien-3,20-dion) | | |
| 4) | Methylprednisolon-21-hemisuccinat-Natrium | 0,1<br>1,0 | 3<br>9 |
| 5) | Methylprednisolon-21-m-sulfobenzoat-Natrium | 0,1<br>1,0 | 0<br>4 |
| 6) | Methylprednisolon-21-(3-sulfopropionat)-Natrium | 0,1<br>1,0 | 5<br>9 |
| | C) Derivate des Diflucortolons (=6α,9α-Difluor-11β,21-dihydroxy-16α-methyl-1,4-pregnadien-3,20-dion) | | |
| 7) | Diflucortolon-21-hemisulfat-Natrium | 0,003<br>0,03 | 0<br>0 |
| 8) | Diflucortolon-21-(3-sulfopropionat)-Natrium | 0,03<br>0,3 | 0<br>10 |
| 9) | Diflucortolon-21-(3-sulfopropionat)-Kalium | 0,03<br>0,3 | 0<br>10 |

Ergebnisse des Endotoxin-Schock-Testes (Fortsetzung)

| Nr. | Substanz | Dosis in mg/kg Tier | Zahl der überlebenden Tiere |
|---|---|---|---|
| | D) Derivate des Triamcinolonacetonids (=9$\alpha$-Fluor-11$\beta$,21-dihydroxy-16$\alpha$,17$\alpha$-isopropylidendioxy-1,4-pregnadien-3,20-dion) | | |
| 10) | Triamcinolonacetonid-21-phosphat-Natrium | 0,03<br>0,3 | 0<br>7 |
| 11) | Triamcinolonacetonid-21-hemisuccinat-Natrium | 0,03<br>0,3 | 6<br>9 |
| 12) | Triamcinolonacetonid-21-(3-sulfopropionat)-Natrium | 0,03<br>0,3 | 6<br>10 |
| | E) Derivate des Prednisolon-(= 11$\beta$,17$\alpha$,21-Trihydroxy-1,4-pregnadien-3,20-dion) | | |
| 13) | Prednisolon-21-sulfoacetat-Natrium | 0,1<br>0,3<br>1,0<br>3,0 | 3<br>4<br>4<br>6 |
| 14) | Prednisolon-21-(3-sulfopropionat)-Natrium | 0,1<br>0,3<br>1,0<br>3,0 | 3<br>6<br>7<br>8 |
| 15) | Prednisolon-21-(4-sulfobutyrat)-Natrium | 0,1<br>0,3<br>1,0<br>3,0 | 2<br>4<br>3<br>6 |

Es sei angemerkt, daß die Wasserlöslichkeit insbesondere der Alkalimetallsalze der neuen Kortikoid-21-sulfopropionate oft erstaunlich groß ist. So lösen sich beispielsweise in einem ml Wasser bei 25°C:

ca. 350 mg Prednisolon-21-(3-sulfopropionat)-Natrium
ca. 350 mg 6$\alpha$-Methylprednisolon-21-(3-sulfopropionat)-Natrium
ca. 500 mg Betamethason-2-(3-sulfopropionat)-Natrium oder
ca. 350 mg Diflucortolon-21-(3-sulfopropionat)-Natrium.

Demzufolge eignen sich diese Verbindungen sehr gut zur Herstellung hochdosierter wässriger Injektionslösungen, welche man für die Behandlung lebensbedrohlicher Schockzustände benötigt.

Demgegenüber ist die Wasserlöslichkeit der Natriumsalze von Kortikoid-21-sulfobenzoaten wesentlich geringer. So lösen sich beispielsweise in einem ml Wasser bei 25°C:

ca. 10 mg Prednisolon-21-(m-sulfobenzoat)-Natrium oder
ca. 3,5 mg Dexamethason-21-(m-sulfobenzoat)-Natrium.

Die Verträglichkeit der neuen Verbindungen hängt im wesentlichen von der Verträglichkeit des daraus systemisch freigesetzten 21-Hydroxykortikoids ab. Die bei der Spaltung freigesetzte 3-Sulfopropionsäure verursacht im angewendeten Dosisbereich keine Nebenwirkungen.

Die neuen Kortikoid-21-sulfopropionate und ihre Salze können in üblicher Weise zu Arzneimittelspezialitäten verarbeitet werden, indem man sie gegebenenfalls mit geeigneten Zusätzen, Trägersubstanzen und Stabilisatoren in die gewünschte Applikationsform: wie Injektionslösungen, Augentropfen, Nasentropfen, Klysmen, Pastillen, Tabletten oder Inhalationslösungen überführt.

Die so erhaltenen Arzneimittelspezialitäten können zur Behandlung solcher Erkrankungen dienen, bei denen üblicherweise eine Therapie mit Kortikoiden zweckmäßig ist. Dies sind beispielsweise:

Schwere allergische Reaktionen, allergischer Schock, akut lebensbedrohliche Zustände, Schock und Kollaps, Status asthmaticus, zerebrales Ödem, Transfusionszwischenfälle, akute Vergiftungen, ausgedehnte Verbrennungen, Quincke Ödem, schwere Stoffwechselstörungen, akute schwere Dermatosen, akute Infektionskrankheiten (Zusatztherapie), Pseudokrupp (bei Kindern), frischer Herzinfarkt, Lungenödem durch Inhalation toxischer Substanzen, akute Nebennierensuffizienz, hyperthyreote Krise, Waterhouse-Fridrichsen-Syndrom, Apoplexie. (Intraartikulär): Polyarthritis rheumatica, akute und chronische Bindegewebeerkrankungen, Arthrosis deformans, allergische, rheumatologische und dermatologische Erkrankungen, die auf eine orale Kortikoidtherapie ansprechen, Kortikoidempfindliche Entzündungen der Mundschleimhaut, allergische Reaktionen, Lichen, Pemphigoide, entzündliche und allergische Erkrankungen des Auges, Iritis, Iridocyclitis, Konjuktivitis, Blepharitis, Erkrankungen der vorderen Uvea, allergische u. chronische Rhinitiden, Rhinitis vasomotorica, nicht eitrige Sinusitis, Heuschnupfen und Colitis ulcerosa.

Die nachfolgenden Beispiele dienen zur näheren Erläuterung der Erfindung.

Beispiel 1

a) Die Lösung von 4,5 g 3-Sulfopropionsäureanhydrid in 150 ml Methylenchlorid wird mit 4,5 g 6$\alpha$-Fluor-11ß,21-dihydroxy-16$\alpha$-methyl-1,4-pregnadien-3,20-dion versetzt und 2 Stunden im Eisbad gerührt. Während dieser Zeit bildet sich ein Niederschlag, der nach dem Abdekantieren der Methylenchlordphase in 100 ml Wasser gelöst wird. Die wässrige Lösung wird nacheinander mit jeweils 100 ml eines Gemisches aus Methylenchlorid und Isopropylalkohol im Verhältnis (9:1), (8:2), (7:3) und (6:4) ausgeschüttelt. Der Extrakt der mit dem Gemisch (7:3) erhalten wird, liefert nach dem Eindampfen im Vakuum 4,2 g 6$\alpha$-Fluor-11$\beta$-hydroxy-16$\alpha$-methyl-21-(3-sulfopropionyloxy)-1,4-pregnadien-3,20-dion.

b) 513 mg 6$\alpha$-Fluor-11$\beta$-hydroxy-16$\alpha$-methyl-21-(3-sulfopropionyloxy)-1,4-pregnadien-3,20-dion werden in 10 ml destilliertem Wasser gelöst und unter Kontrolle des pH-Wertes mittels Glaselektrode mit wässriger 0,1 N-Natriumhydroxidlösung bis zum pH-Wert von 7,0 titriert. Die Salzlösung wird 3 mal mit je 50 ml Diethylether ausgeschüttelt und anschließend im Vakuum bei 0,1 mbar gefriergetrocknet. Man erhält 496 mg Natriumsalz des 6$\alpha$-Fluor-11$\beta$-hydroxy-16$\alpha$-methyl-21-(3-sulfopropionyloxy)-1,4-pregnadien-3,20-dions in Form eines lockeren weißen Pulvers. Schmelzpunkt 190—200°C. $[\alpha]_D^{25} = 94°$ (Methanol) UV: $\varepsilon_{242} = 15700$ (Methanol).

Beispiel 2

802 mg 6$\alpha$-Fluor-11$\beta$-hydroxy-16$\alpha$-methyl-21-(3-sulfopropionyloxy)-1,4-pregnadien-3,20-dion werden mit wässriger 0,1 N-Kaliumhydroxidlösung, unter den im Beispiel 1 b angegebenen Bedingungen, in das Kaliumsalz überführt. Ausbeute 698 mg. Schmelzpunkt 190—200°C $[\alpha]_D^{25} = +94°$ (Wasser). UV: $\varepsilon_{242} = 14200$ (Methanol).

Beispiel 3

461 mg 6$\alpha$-Fluor-11$\beta$-hydroxy-16$\alpha$-methyl-21-(3-sulfopropionyloxy)-1,4-pregnadien-3,20-dion werden mit wässriger 0,1 Lithiumhydroxidlösung, unter den im Beispiel 1 b angegebenen Bedingungen, in das Lithiumsalz überführt. Ausbeute 370 mg. Schmelzpunkt 200—210°C $[\alpha]_D^{25} = +99°$ (Wasser).

Beispiel 4

513 mg 6$\alpha$-Fluor-11$\beta$-hydroxy-16$\alpha$-methyl-21-(3-sulfopropionyloxy)-1,4-pregnadien-3,20-dion werden mit wässriger 0,1 N Ammoniumhydroxidlösung, unter den im Beispiel 1 b angegebenen Bedingungen in das Ammoniumsalz überführt. Ausbeute 440 mg. Schmelzpunkt 169—176°C $[\alpha]_D^{25} = +100°$ (Wasser). UV: $\varepsilon_{242} = 14900$ (Methanol).

Beispiel 5

a) 4,5 g 6$\alpha$,9-Difluor-11$\beta$-dihydroxy-16$\alpha$-methyl-1,4-pregnadien-3,20-dion werden, unter den im Beispiel 1 a angegebenen Bedingungen in 6$\alpha$,9-Difluor-11$\beta$-hydroxy-16$\alpha$-methyl-21-(3-sulfopropionyloxy)-1,4-pregnadien-3,20-dion überführt. Ausbeute 3,32 g (mit Diethylether verrieben). Schmelzpunkt 222—227° (unter Zersetzung).

b) Unter den im Beispiel 1 b beschriebenen Bedingungen werden 1,06 g 6$\alpha$,9-Difluor-11$\alpha$-hydroxy-16$\alpha$-methyl-21-(3-sulfopropionyloxy)-1,4-pregnadien-3,20-dion in das Natriumsalz überführt. Ausbeute 870 mg. Schmelzpunkt 200—225°C $[\alpha]_D^{25} = +94°$ (Methanol). UV: $\varepsilon_{238} = 16000$ (Methanol).

Beispiel 6

796 mg 6$\alpha$,9-Difluor-11$\beta$-hydroxy-16$\alpha$-methyl-21-(3-sulfopropionyloxy)-1,4-pregnadien-3,20-dion werden, unter den im Beispiel 1 b angegebenen Bedingungen, mit wässriger 0,1 N Kaliumhydroxidlösung in das Kaliumsalz überführt. Ausbeute 800 mg. Schmelzpunkt 207—215°C $[\alpha]_D^{25} = +98°$ (Wasser). UV: $\varepsilon_{238} = 15900$ (Methanol).

Beispiel 7

530 mg 6$\alpha$,9-Difluor-11$\beta$-hydroxy-16$\alpha$-methyl-21-(3-sulfopropionyloxy)-1,4-pregnadien-3,20-dion werden mit wässriger 0,1 N Lithiumhydroxidlösung unter den im Beispiel 1 b angegebenen Bedingungen in das Lithiumsalz überführt. Ausbeute 498 mg. Schmelzpunkt 210—220°C $[\alpha]_D^{25} = +98°$ (Wasser). UV: $\varepsilon_{238} = 15400$ (Methanol).

Beispiel 8

638 mg 6$\alpha$,9-Difluor-11$\beta$-hydroxy-16$\alpha$-methyl-21-(3-sulfopropionyloxy)-1,4-pregnadien-3,20-dion werden mit wässriger 1 N Ammoniumhydroxidlösung, unter den im Beispiel 1 b angegebenen Bedingungen in das Ammoniumsalz überführt. Ausbeute 578 mg. Schmelzpunkt 168—184°C $[\alpha]_D^{25} = +104°$ (Wasser). UV: $\varepsilon_{238} = 16000$ (Methanol).

Beispiel 9

531 mg 6$\alpha$9-Difluor-11$\beta$-hydroxy-16$\alpha$-methyl-21-(3-sulfopropionyloxy)-1,4-pregnadien-3,20-dion werden mit wässriger 0,04 N Calciumhydroxidlösung, unter den im Beispiel 1 b beschriebenen Bedingungen in das Calciumsalz überführt. Ausbeute 456 mg. $[\alpha]_D^{25} = +93°$ (Wasser). UV: $\varepsilon_{238} = 28200$ (Methanol).

Beispiel 10

A. 4,5 g 11$\beta$,17,21-Trihydroxy-1,4-pregnadien-3,20-dion unter den im Beispiel 1—A angegebenen Bedingungen, in 11$\beta$,17-Dihydroxy-21-(3-sulfopropionyloxy)-1,4-pregnadien-3,20-dion überführt. Ausbeute 2,28 g.

B. 680 mg 11$\beta$,17-Dihydroxy-21-(3-sulfopropionyloxy)-1,4-pregnadien-3,20-dion werden, unter den im Beispiel 1—B beschriebenen Bedingungen, in das Natriumsalz überführt. Ausbeute 500 mg. Schmelzpunkt 180—200°C (unter Zersetzung). $[\alpha]_D^{25} = +79°$ (Methanol). UV: $\varepsilon_{243} = 10600$ (Methanol).

Beispiel 11

A. 1.0 g 11$\beta$,17,21-Trihydroxy-6$\alpha$-methyl-1,4-pregnadien-3,20-dion werden, unter den im Beispiel 1—A beschriebenen Bedingungen in 11$\beta$,17-Dihydroxy-6$\alpha$-methyl-21-(3-sulfopropionyloxy)-1,4-pregnadien-3,20-dion überführt. Ausbeute 600 mg.

B. 580 mg 11$\beta$,17-Dihydroxy-6$\alpha$-methyl-21-(3-sulfopropionyloxy)1,4-pregnadien-3,20-dion werden, unter den im Beispiel 1—B beschriebenen Bedingungen, in das Natriumsalz überführt. Ausbeute 470 mg. Schmelzpunkt 206—224°C $[\alpha]_D^{25} = +87°$ (Wasser). UV: $\varepsilon_{243} = 13100$ (Methanol).

Beispiel 12

A. 3,0 g 9-Fluor-11$\beta$,21-dihydroxy-16$\alpha$-17-isopropylidendioxy-1,4-pregnadien-3,20-dion werden, unter den im Beispiel 1—A beschriebenen Bedingungen, in 9-Fluor-11$\beta$-hydroxy-16$\alpha$,17-isopropylidendioxy-21-(3-sulfopropionyloxy)-1,4-pregnadien-3,20-dion überführt. Ausbeute 2,1 g.

B. 742 mg 9-Fluor-11$\beta$-hydroxy-16$\alpha$,17-isopropylidendioxy-21-(3-sulfopropionyloxy)-1,4-pregnadien-3,20-dion werden, unter den im Beispiel 1—B beschriebenen Bedingungen, in das Natriumsalz überführt. Ausbeute 704 mg. Schmelzpunkt 238—243°C $[\alpha]_D^{25} = +93°$ (Wasser). UV: $\varepsilon_{238} = 14100$ (Methanol).

Beispiel 13

A. 2,5 g 9-Fluor-11$\beta$17,21-trihydroxy-16$\alpha$-methyl-1,4-pregnadien-3,20-dion werden, unter den im Beispiel 1—A beschriebenen Bedingungen, in 9-Fluor-11$\beta$,17-dihydroxy-16$\alpha$-methyl-21-(3-sulfopropionyloxy)-1,4-pregnadien-3,20-dion überführt. Ausbeute 992 mg.

B. 950 mg 9-Fluor-11$\beta$17-dihydroxy-16$\alpha$-methyl-21-(3-sulfopropionyloxy)-1,4-pregnadien-3,20-dion werden, unter den im Beispiel 1—B angegebenen Bedingungen, in das Natriumsalz überführt. Ausbeute 690 mg. Schmelzpunkt 215—230°C $[\alpha]_D^{25} = +81°$ (Wasser). UV: $\varepsilon_{235} = 14200$ (Methanol).

Beispiel 14

a) 2,5 9-Chlor-6$\alpha$-fluor-11$\beta$,21-dihydroxy-16$\alpha$-methyl-1,4-pregnadien-3,20-dion werden unter den im Beispiel 1 a beschriebenen Bedingungen in 9-Chlor-6$\alpha$-fluor-11$\beta$-hydroxy-16$\alpha$-methyl-21-(3-sulfopropionyloxy)-1,4-pregnadien-3,20-dion überführt. Ausbeute 1,96 g (nach dem Verreiben mit Diethylether).

b) 547 mg 9-Chlor-6$\alpha$-fluor-11$\beta$-hydroxy-16$\alpha$-methyl-21-(3-sulfopropionyloxy)-1,4-pregnadien-3,20-dion werden unter den im Beispiel 1 b beschriebenen Bedingungen in das Natriumsalz überführt. Ausbeute 508 mg. Schmelzpunkt 201—230°C (unter Zersetzung). $[\alpha]_D^{25} = +122°$ (Wasser). UV: $\varepsilon_{237} = 15000$ (Methanol).

Beispiel 15

a) 1,1 g 9-Fluor-11$\beta$21-dihydroxy-16$\alpha$-methyl-1,4-pregnadien-3,20-dion werden unter den im Beispiel 1 a beschriebenen Bedingungen in 9-Fluor-11$\beta$-hydroxy-16$\alpha$-methyl-21-(3-sulfopropionyloxy)-1,4-pregnadien-3,20-dion überführt. Ausbeute 460 mg.

B. 460 mg 9-Fluor-11β-hydroxy-16α-methyl-21-(3-sulfopropionyloxy)-1,4-pregnadien-3,20-dion werden, unter den im Beispiel 1—B angegebenen Bedingungen, in das Natriumsalz überführt. Ausbeute 427 mg. Schmelzpunkt 189—200°C $[\alpha]_D^{25} = +103°$ (Wasser). UV: $\varepsilon_{239} = 14300$ (Methanol).

Beispiel 16

a) 2,0 g 9-Fluor-11β17,21-trihydroxy-16β-methyl-1,4-pregnadien-3,20-dion werden unter den im Beispiel 1 a beschriebenen Bedingungen in 9-Fluor-11β,17-dihydroxy-16β-methyl-21-(3-sulfopropionyloxy)-1,4-pregnadien-3,20-dion überführt. Ausbeute 970 mg.

b) 970 mg 9-Fluor-11β17-dihydroxy-16β-methyl-21-(3-sulfopropionyloxy)-1,4-pregnadien-3,20-dion werden unter den im Beispiel 1 b angegebenen bedingungen in das Natriumsalz überführt. Ausbeute 742 mg. Schmelzpunkt 205—220°C $[\alpha]_D^{25} = +82°$ (Wasser). UV: $\varepsilon_{238} = 13200$ (Methanol).

Beispiel 17

a) 2,0 g 11β,17,21-Trihydroxy-4-pregnen-3,20-dion werden unter den im Beispiel 1 a beschriebenen Bedingungen in 11β,17-Dihydroxy-21-(3-sulfopropionyloxy)-1,4-pregnen-3,20-dion überführt. Ausbeute 520 mg.

b) 520 mg 11β,17-Dihydroxy-21-(3-sulfopropionyloxy)-4-pregnen-3,20-dion werden unter den im Beispiel 1 b beschriebenen Bedinungen in das Natriumsalz überführt. Ausbeute 382 mg. $[\alpha]_D^{25} = +123°$ (Wasser). UV: $\varepsilon_{243} = 14400$ (Methanol). Schmelzpunkt 192—210°C.

Beispiel 18

a) 1,0 g 9-Fluor-11β,17,21-trihydroxy-4-pregnen-3,20-dion werden unter den im Beispiel la beschriebenen Bedingungen in 9-Fluor-11β,17-dihydroxy-21-(3-sulfopropionyloxy)-4-pregnen-3,20-dion überführt. Ausbeute 900 mg.

b) 900 mg 9-Fluor-11β,17-dihydroxy-21-(3-sulfopropionyloxy)-4-pregnen-3,20-dion werden unter den im Beispiel 1 b beschriebenen Bedingungen in das Natriumsalz überführt. Ausbeute 610 mg. Schmelzpunkt 205—225°C $[\alpha]_D^{25} = +109°$ (Wasser). UV: $\varepsilon_{239} = 14300$ (Methanol).

Beispiel 19

a) 2,5 g 9-Fluor-11β,21-dihydroxy-16β-methyl-17-valeryloxy-1,4-pregnadien-3,20-dion werden unter den im Beispiel 1a beschriebenen Bedingungen in 9-Fluor-11β-hydroxy-16β-methyl-21-(3-sulfopropionyloxy)-17-valeryloxy-1,4-pregnadien-3,20-dion überführt. Ausbeute 2,37 g.

b) 890 mg 9-Fluor-11β-hydroxy-16β-methyl-21-(3-sulfopropionyloxy)-17-valeryloxy-1,4-pregnadien-3,20-dion werden unter den im Beispiel 1 b beschriebenen Bedingungen in das Natriumsalz überführt. Ausbeute 550 mg. Schmelzpunkt 190—198°C $[\alpha]_D^{25} = +81°$ (Wasser). UV: $\varepsilon_{239} = 14600$ (Methanol).

Beispiel 20

1,4 g 9-Fluor-11β-hydroxy-16β-methyl-21-(3-sulfopropionyloxy)-17-valeryloxy-1,4-pregnadien-3,20-dion werden mit wässriger 0,1 N Kaliumhydroxidlösung unter den im Beispiel 1 b angegebenen Bedingungen in das Kaliumsalz überführt. Ausbeute 960 mg. Schmelzpunkt 196—200°C $[\alpha]_D^{25} = +78°$ (Wasser). UV: $\varepsilon_{240} = 14900$ (Methanol).

Beispiel 21

A. 1.9 g 17-Butyryloxy-11β,-21-dihydroxy-4-pregnen-3,20-dion werden, unter den im Beispiel 1—A beschriebenen Bedingungen, in 17-Butyryloxy-11β-hydroxy-21-(3-sulfopropionyloxy)-4-pregnen-3,20-dion überführt. Ausbeute 600 mg.

B. 500 mg 17-Butyryloxy-11β-hydroxy-21-(3-sulfopropionyloxy)-4-pregnen-3,20-dion werden, unter den in Beispiel 1—B beschriebnen Bedingungen, in das Natriumsalz überführt. Ausbeute 300 mg. Schmelzpunkt 230—253°C (unter Zersetzung). $[\alpha]_D^{25} = +68°$ (Wasser). UV: $\varepsilon_{239} = 15800$ (Methanol).

Beispiel 22

A. 1.0 g 6α-Fluor-11β,17,21-trihydroxy-1,4-pregnadien-3,20-dion werden, unter den im Beispiel 1—A angegebenen Bedingungen, in 6α-Fluor-11β,17-dihydroxy-21-(3-sulfopropionyloxy)-1,4-pregnadien-3,20-dion überführt. Ausbeute 590 mg.

B. 500 mg 6α-Fluor-11β,17 dihydroxy-21-(3-sulfopropionyloxy)-1,4-pregnadien-3,20-dion werden, unter den im Beispiel 1—B angegebenen Bedingungen, in das Natriumsalz überführt. Ausbeute 450 mg. Schmelzpunkt 230—278°C (unter Zersetzung). $[\alpha]_D^{25} = +91°$ (Wasser). UV: $\varepsilon_{240} = 13800$ (Methanol).

Beispiel 23

A. 490 mg 11β,17a,21-Trihydroxy-D-homo-1,4-pregnadien-3,20-dion werden, unter den in Beispiel 1—A angegebenen Bedingungen, in 11β,17a-Dihydroxy-21-(3-sulfopropionyloxy)-D-homo-1,4-pregnadien-3,20-dion überführt. Ausbeute 290 mg.

B. 290 mg 11$\beta$,17a-Dihydroxy-21-(3-sulfopropionyloxy)-D-homo-1,4-pregnadien-3,20-dion werden, unter den im Beispiel 1—B angegebenen Bedingungen, in das Natriumsalz überführt. Ausbeute 250 mg. Schmelzpunkt 220—274°C (unter Zersetzung). $[\alpha]_D^{25} = +92°$ (Methanol). UV: $\varepsilon_{244} = 10700$ (Methanol).

Beispiel 24

A. 495 mg 9-Fluor-11$\beta$,17a,21-Trihydroxy-D-homo-1,4-pregnadien-3,20-dion werden, unter den im Beispiel 1—A angegebenen Bedingungen, in 9-Fluor-11$\beta$,17a-dihydroxy-21-(3-sulfopropionyloxy)-D-homo-1,4-pregnadien-3,20-dion überführt. Ausbeute 440 mg.

B. 440 mg 9-Fluor-11$\beta$,17a-dihydroxy-21-(3-sulfopropionyloxy)-D-homo-1,4-pregnadien-3,20-dion werden, unter den im Beispiel 1—B angegebenen Bedingungen, in das Natriumsalz überführt. Ausbeute 370 mg. Schmelzpunkt 235—283°C (unter Zerset zung). $[\alpha]_D^{25} = +91°$ (Methanol). UV: $\varepsilon_{240} = 13400$ (Methanol).

Beispiel 25

A. 500 mg 6-Chlor-11$\beta$,17,21-trihydroxy-1,4,6-pregnatrien-3,20-dion werden, unter den im Beispiel 1—A beschriebenen Bedingungen, in 6-Chlor-11$\beta$,17-dihydroxy-21-(3-sulfopropionyloxy)-1,4,6-pregnatrien-3,20-dion überführt. Ausbeute 380 mg.

B. 380 mg 6-Chlor-11$\beta$,17-dihydroxy-21-(3-sulfopropionyloxy)-1,4-6-pregnatrien-3,20-dion werden, unter den im Beispiel 1—B angegebenen Bedingungen, in das Natriumsalz überführt. Ausbeute 350 mg. Schmelzpunkt 190—240°C (unter Zersetzung). $[\alpha]_D^{25} = +48°$ (Methanol). UV: $\varepsilon_{227} = 9100$, $\varepsilon_{255} = 8100$, $\varepsilon_{298} = 8000$ (Methanol).

Beispiel 26

A. Ein Lösung von 10 g 6-Chlor-17-hydroxy-1$\alpha$,2$\alpha$-methylen-4,6-pregnadien-3,20-dion in 75 ml Methanol und 75 ml Tetrahydrofuran wird mit 15 g Calciumoxid und 500 mg Azoisobutyronitril versetzt. Man tropft einige ml einer Lösung von 10 g Jod in 50 ml Tetrahydrofuran und 30 ml Methanol hinzu. Nach einer Induktionszeit von 60 Minuten entfärbt sich die Lösung, die restliche Jodlösung wird innerhalb 8 Stunden eingetropft. Das Reaktionsgemisch wird mit 500 ml Dichlormethan verdünnt, das Calciumoxid wird abfiltriert und das Filtrat mit Natriumthiosulfatlösung und Wasser gewaschen, über Natriumsulfat getrocknet und bei 35°C im Vakuum eingeengt. Man erhält 13 g 6-Chlor-17-hydroxy-21-jod-1$\alpha$,2$\alpha$-methylen-4,6-pregnadien-3,20-dion. Dieses wird in 130 ml Aceton und 45 ml Essigsäure gelöst, mit 69 ml Triethylamin versetzt und 90 Minuten unter Rückfluß erhitzt. Die Lösung wird in Eiswasser eingerührt, der entstehende Niederschlag isoliert und an Kieselgel chromatographiert. Mit 16—20% Aceton-Pentan erhält man 6.1 g 21-Acetoxy-6-chlor-17-hydroxy-1$\alpha$,2$\alpha$-methylen-4,6-pregnadien-3,20-dion vom Schmelzpunkt 224°C.

B. Ein 2 1-Erlenmeyerkolben, der 500 ml einer 30 Minuten bei 120°C im Autoklaven sterilisierten Nährlösung aus 1% Cornsteep liquor, 1% Sojapuder und 0,005% Sojaöl, eingestellt auf pH 6.2, enthält, wird mit einer Lyophilkultur von Curvularia lunata (NRRL 2380) beimpft und 72 Stunden bei 30°C auf einem Rotationsschüttler geschüttelt. Mit dieser Vorkultur wird dann ein 20 l -Fermenter beimpft, der 15 l eines bei 121°C und 1,1 atü sterilisierten Mediums aus 1% Cornsteep liquor, 0,5% Stärkezucker und 0,005% Sojaöl, eingestellt auf pH 6.2, enthält. Unter Zugabe von Silicon SH als Antischaummittel wird bei 29°C unter Belüftung (10 l/Min.) 0,7 atü Druck und Rühren (220 U/Min.) 24 Stunden germiniert. 1 Liter der Kulturbrühe wird unter sterilen Bedingungen in 14 l eines wie oben sterilisierten Mediums aus 1% Cornsteep liquor, 1.25% Sojapuder und 0,005% Sojaöl überführt und unter gleichen Bedingungen angezüchtet. Nach 12 Stunden wird eine Lösung von 15 g 21-Acetoxy-6-chlor-17-hydroxy-1$\alpha$,2$\alpha$-methylen-4,6-pregnadien-3,20-dion in 150 ml Dimethylformamid zugegeben und weiter gerührt und belüftet. Nach 26 Stunden Kontaktzeit wird der Fermenterinhalt zweimal mit je 10 l Methylisobutylketon ausgerührt und die vereinigten Extrakte bei 50°C Badtemperatur im Vakuum eingedampft. Der Rückstand wird in Methanol aufgenommen, das nicht gelöste Siliconöl abgetrennt, die Lösung mit A-Kohle behandelt und nach dem Konzentrieren zur Kristallisation gebracht. Das ausgeschiedene kristalline Produkt wurde zur weiteren Reinigung über eine Kieselgelsäule mittels eines Methylenchlorid-Aceton-Gradienten chromatographiert und danach aus Aceton-Methanol umkristallisiert. Das reine 6-Chlor-11$\beta$,17,21-trihydroxy-1$\alpha$,2$\alpha$-methylen-4,6-pregnadien-3,20-dion (8,1 g) schmilzt bei 271—272°C. UV: $\varepsilon_{283} = 17300$ (Methanol). $[\alpha]_D^{25} = +273°$ (Methanol).

C. 1.09 g 6-Chlor-11$\beta$,17,21-trihydroxy-1$\alpha$,2$\alpha$methylen-4,6-pregnadien-3,20-dion werden, unter den im Beispiel 1—A angegebenen Bedingungen, in 6-Chlor-11$\beta$,17-dihydroxy-1$\alpha$,2$\alpha$-methylen-21-(3-sulfopropionyloxy)-4,6-pregnadien-3,20-dion überführt. Ausbeute 620 mg.

D. 620 mg 6-Chlor-11$\beta$,17-dihydroxy-1$\alpha$,2$\alpha$-methylen-21-(3-sulfopropionyloxy)-4,6-pregnadien-3,20-dion werden, unter den im Beispiel 1—B angegebenen Bedingungen, in das Natriumsalz überführt. Ausbeute 260 mg. Schmelzpunkt 238—281°C (unter Zersetzung). $[\alpha]_D^{25} = +194°$ (Methanol). UV: $\varepsilon_{283} = 14000$ (Methanol).

Galenische Zubereitungen

Beispiel 1

Zusammensetzung von Augentropfen:

100 mg 9-Fluor-11β,17-dihydroxy-16β-methyl-21-(3-sulfopropionyloxy)-1,4-pregnadien-3,20-dion-Natrium,

2 mg Benzalkoniumchlorid und

700 mg Natriumchlorid werden in 100 ml Wasser für Injektionszwecke gelöst. Die Lösung wird sterilisiert und unter aseptischen Bedingungen abgefüllt.

Beispiel 2

Zusammensetzung von Ohrentropfen:

0,2 g 6α,9-Difluor-11β-hydroxy-16α-methyl-21-(3-sulfopropionyloxy)-1,4-pregnadien-3,20-dion-Natrium,

1,5 g Chloramphenicol,

98,3 g Propan-1,2-diol.

Beispiel 3

Zusammensetzung von Nasentropfen:

25 mg 9-Fluor-11β,17-dihydroxy-16α-methyl-21-(3-sulfopropionyloxy)-1,4-pregnadien-3,20-dion-Natrium und

50 mg Oxymetazolin-Hydrochlorid werden in 100 ml Aqua bidestillata gelöst. Die Lösung wird sterilisiert und unter aseptischen Bedingungen abgefüllt.

Beispiel 4

Lösungen zur intravenösen Injektion:

A. 10,0 g 11β,17-Dihydroxy-6α-methyl-21-(3-sulfopropionyloxy)-1,4-pregnadien-3,20-dion-Natrium werden in 100 ml Wasser für Injektionszwecke gelöst. Die Lösung wird klar filtriert und in Ampullen abgefüllt. Die verschlossenen Ampullen werden 30 minuten in gespanntem Wasserdampf auf 115°C erhitzt.

B. 5,0 g 6α-Fluor-11β-hydroxy-16α-methyl-21-(3-sulfopropionyloxy)-1,4-pregnadien-3,20-dion-Kalium werden in 100 ml Wasser für Injektionszwecke gelöst. Die Lösung wird, wie vorstehend beschrieben, abgefüllt und sterilisiert.

Beispiel 5

Zusammensetzung einer Tablette zur oralen Applikation:

5,00 mg 11β,17-Dihydroxy-21-(3-sulfopropionyloxy)-1,4-pregnadien-3,20-dion-Natrium,
71,47 mg Maisstärke, DAB 7
36,00 mg Lactose, DAB 7
6,00 mg Talkum, DAB 7
1,50 mg Gelatine, weiß, DAB 7
0,02 mg p-Hydroxybenzoesäuremethylester, DAB 7
0,01 mg p-Hydroxybenzoesäurepropylester, DAB 7.

**Patentansprüche**

1. Kortikoid-21-sulfopropionate der allgemeinen Formel I

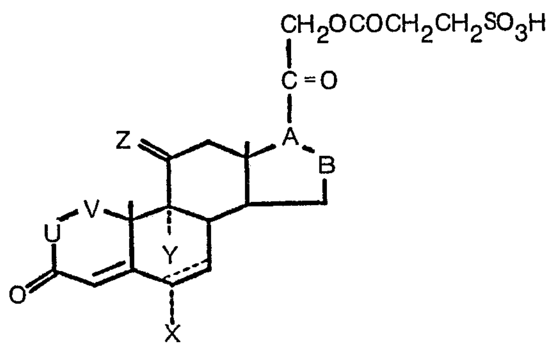

(I)

worin

..... eine Einfachbindung oder eine Doppelbindung

X ein Wasserstoffatom, ein Chloratom, ein Fluoratom oder eine Methylgruppe,

Y ein Wasserstoffatom, ein Fluoratom oder ein Chloratom,

Z eine Oxogruppe oder ein α-ständiges Wasserstoffatom und eine β-ständige Hydroxygruppe —U—V— die Gruppierungen $—CH_2—CH_2—$,

CH=CH oder 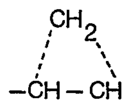 und 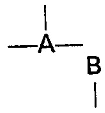 die Gruppierungen

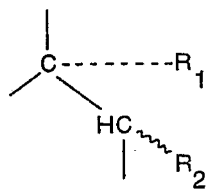 , 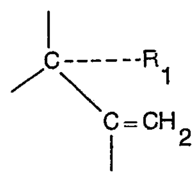 , 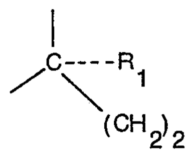 oder 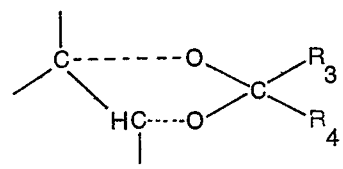

mit $R_1$ in der Bedeutung eines Wasserstoffatoms, einer Hydroxygruppe oder einer 1 bis 8 Kohlenstoffatome enthaltenden Acyloxygruppe,
$R_2$ in der Bedeutung eines Wasserstoffatoms oder einer Methylgruppe und
$R_3$ und $R_4$ in der Bedeutung einer 1 bis 4 Kohlenstoffatome enthaltenden Alkylgruppe darstellen, und deren Salze mit physiologisch unbedenklichen Basen.

2. Kortikoid-21-sulfopropionate der allgemeinen Formel gemäß Anspruch 1, dadurch gekennzeichnet, daß ..... eine Doppelbindung, X ein Fluoratom, Y ein Wasserstoffatom, ein Fluoratom oder ein Chloratom, Z ein α-ständiges Wasserstoffatom und eine β-ständige Hydroxygruppe und

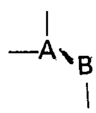 die Gruppierung 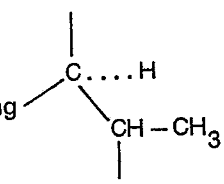

darstellen und deren Salze mit physiologisch unbekden-klichen Basen.

3. Kortikoid-21-sulfopropionate der allgemeinen Formel I gemäß Anspruch 1, dadurch gekennzeichnet, daß ..... eine Doppelbindung, X ein Wasserstoffatom, Y ein Fluoratom oder ein Chloratom, Z ein α-ständiges Wasserstoffatom und eine β-ständige Hydroxygruppe und

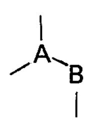 die Gruppierung 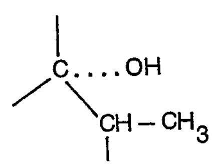

darstellen und deren Salze mit physiologisch unbedenklichen Basen.

4. Kortikoid-21-sulfopropionate der allgemeinen Formel I gemäß Anspruch 1, dadurch gekennzeichnet, daß ..... eine Doppelbindung, X ein Wasserstoffatom oder ein Fluoratom,
Y ein Wasserstoffatom oder ein Fluoratom,
Z eine α-ständiges Wasserstoffatom und eine β-ständige Hydroxygruppe und

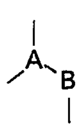 die Gruppierung 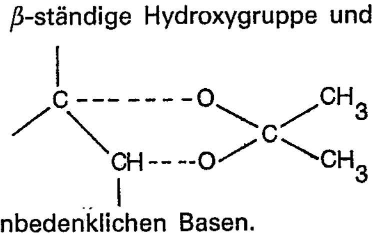

darstellen und deren Salze mit physioligisch unbedenklichen Basen.

5. Kortikoid-21-sulfopropionate der allgemeinen Formel I gemäß Anspruch 1, dadurch gekennzeichnet, daß ..... eine Einfachbindung oder eine Doppelbindung, X ein Wasserstoffatom oder eine Methylgruppe, Y ein Wasserstoffatom, Z ein α-ständiges Wasserstoffatom und eine β-ständige Hydroxygruppe und

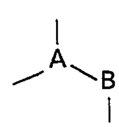 die Gruppierung 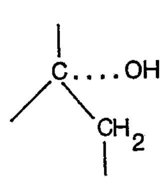

darstellen und deren Salze mit physiologisch unbedenklichen Basen.

6. Kortikoid-21-sulfopropionate der allgemeinen Formel I gemäß Anspruch 1, dadurch gekennzeichnet, daß —A—B— die Gruppierung

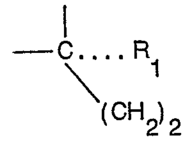

darstellt.

7. Lithium-, Natrium-, Kalium-, Calzium- und Ammoniumsalze von Kortikoid-21-sulfopropionaten der allgemeinen Formel I und II gemäß Anspruch 1 bis 6.

8. Verfahren zur Herstellung von Kortikoid-21-sulfopropionate der allgemeinen Formel I gemäß Patentanspruch 1, dadurch gekennzeichnet, daß man ein Kortikoid der allgemeinen Formel II

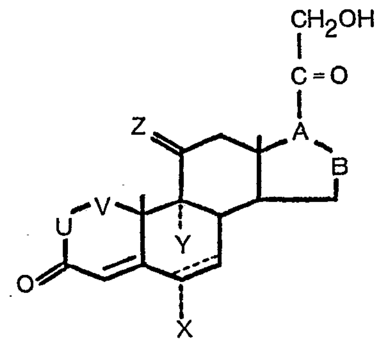

(II),

worin ..... —U—V—, X,Y,Z und —B—A— die im Anspruch 1 genannte Bedeutung besitzen, mit 3-Sulfopropionsäureanhydrid der Formel III

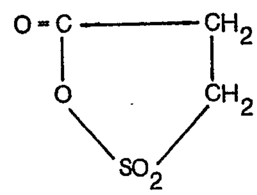

(III),

umsetzt und die erhaltenen Kortikoid-21-sulfopropionate gegebenenfalls mit physiologisch unbedenklichen Basen in ihre Salze überführt.

9. Pharmazeutische Präparate, gekennzeichnet durch den Gehalt an einem Salz eines Kortikoid-21-sulfopropionates gemäß Anspruch 1 bis 8.

10. 6α-Fluor-11β-hydroxy-16α-methyl-21-(3-sulfopropionyloxy)-1,4-pregnadien-3,20-dion und dessen Natrium-, Kalium-, Lithium- und Ammoniumsalze.

11. 6α,9α-Difluor-11β-hydroxy-16α-methyl-21-(3-sulfopropionyloxy)-1,4-pregnadien-3,20-dion und dessen Natrium-, Kalium-, Lithium-, Calzium- und Ammoniumsalze.

12. 11β,17α-Dihydroxy-21-(3-sulfopropionyloxy)-1,4-pregnadien-3,20-dion und dessen Natriumsalz.

13. 11β,17α-Dihydroxy-6α-methyl-21-(3-sulfopropionyloxy)-1,4-pregnadien-3,20-dion und dessen Natriumsalz.

14. 9α-Fluor-11β-hydroxy-16α,17α-isopropylidendioxy-21-(3-sulfopropionyloxy)-1,4-pregnadien-3,20-dion und dessen Natriumsalz.

15. 9α-Fluor-11β,17α-dihydroxy-16α-methyl-21-(3-sulfopropionyloxy)-1,4-pregnadien-3,20-dion und dessen Natriumsalz.

16. 9α-Chlor-6α-fluor-11β-hydroxy-16α-methyl-21-(3-sulfopropionyloxy)-1,4-pregnadien-3,20-dion und dessen Natriumsalz.

17. 9α-Fluor-11β-hydroxy-16α-methyl-21-(3-sulfopropionyloxy)-1,4-pregnadien-3,20-dion und dessen Natrium-salz.

18. 9α-Fluor-11β,17α-dihydroxy-16β-methyl-21-(3-sulfopropionyloxy)-1,4-pregnadien-3,20-dion und dessen Natriumsalz.

19. 11β,17α-Dihydroxy-21-(3-sulfopropionyloxy)-1,4-pregnen-3,20-dion und dessen Natriumsalz.

20. 9α-Fluor-11β-hydroxy-16β-methyl-17α-valeryloxy-21-(3-sulfopropionyloxy)-1,4-pregnadien-3,20-dion, dessen Natrium- und Kaliumsalz.

21. 9α-Fluor-11β,17α-dihydroxy-21-(3-sulfopropionyloxy)-4-pregnen-3,20-dion und dessen Natriumsalz.

22. 17α-Butyryloxy-11β-hydroxy-21-(3-sulfopropionyloxy)-4-pregnen-3,20-dion und dessen Natriumsalz.

23. 6α-Fluor-11β,17α-dihydroxy-21-(3-sulfopropionyloxy)-1,4-pregnadien-3,20-dion und dessen Natriumsalz.

24. 11β,17aα-Dihydroxy-21-(3-sulfopropionyloxy)-D-homo-1,4-pregnadien-3,20-dion und dessen Natriumsalz.

25. 9α-Fluor-11β,17aα-dihydroxy-21-(3-sulfopropionyloxy)-D-homo-1,4-pregnadien-3,20-dion und dessen Natriumsalz.

26. 6-Chlor-11β,17α-dihydroxy-21-(3-sulfopropionyloxy)-1,4,6-pregnatrien-3,20-dion und dessen Natriumsalz.

27. 6-Chlor-11β,17α-dihydroxy-1α,2α-methylen-21-(3-sulfopropionyloxy)-4,6-pregnadien-3,20-dion und dessen Natriumsalz.

**Revendications**

1. Sulfopropionates en 21 de corticoïdes répondant à la formule générale I

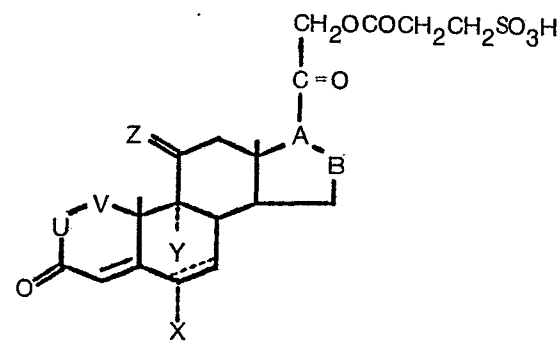

(I),

dans laquelle
.... représente une liaison simple ou une liaison double,
X représente un atome d'hydrogène, de chlore ou de fluor ou un radical méthyle,
Y représente un atome d'hydrogène, de fluor ou de chlore,
Z représente un group oxo, ou un atome d'hydrogène ayant la configuration α et un groupe hydroxy ayant la configuration β,
—U—V— représente l'un des groupements $—CH_2—CH_2—$, —CH=CH— et

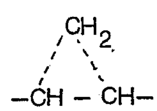

et —A—B représente l'un des groupements:

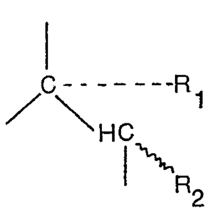

,

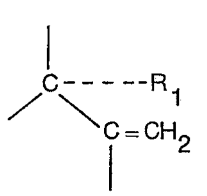

,

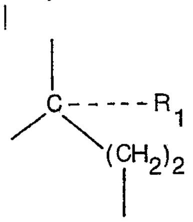

et

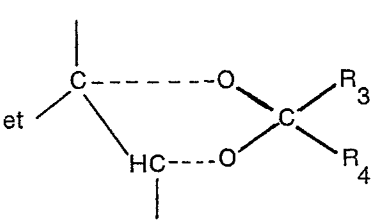

dans lesquels:
$R_1$ représente un atome d'hydrogène, un groupe hydroxy ou un radical acyloxy contenant de 1 à 8 atomes de carbone,
$R_2$ représente un atome d'hydrogène ou un radical méthyle et
$R_3$ et $R_4$ représentent chacun un radical alkyle contenant de 1 à 4 atomes de carbone, ainsi que les sels qu'ils forment avec des bases acceptables du point de vue physioligique.

2. Sulfopropionates en 21 de corticoïdes qui répondent à la formule générale I selon la revendication 1 et qui sont caractérisés en ce que .... représente un double liaison, X représente un atome de fluor, Y représente un atome d'hydrogène, de fluor ou de chlore, Z représente un atome d'hydrogène ayant la configuration α et un groupe hydroxy ayant la configuration β et

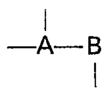 représente le groupement 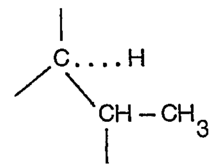

ainsi que les sels qu'ils forment avec des bases acceptables du point de vue physiologique.

3. Sulfopropionates en 21 de corticoïdes qui répondent à la formule générale I selon la revendication 1 et qui sont caractérisés en ce que .... représente une double liaison, X représente un atome d'hydrogène, Y représente un atome de fluor ou de chlore, Z représente un atome d'hydrogène ayant la configuration $\alpha$ ou un groupe hydroxy ayant la configuration $\beta$ et

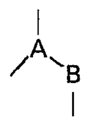 représente le groupement 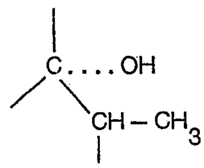 ,

ainsi que les sels qu'ils forment avec des bases physiologiquement acceptables.

4. Sulfopropionates en 21 de corticoïdes qui répondent à la formule générale I selon la revendication 1 et qui sont caractérisés en ce que .... représente un double liaison, X représente un atome d'hydrogène ou de fluor, Y représente un atome d'hydrogène ou de fluor, Z représente un atome d'hydrogène ayant la configuration $\alpha$ et un groupe hydroxy ayant la configuration $\beta$, et

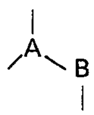 représente le groupement 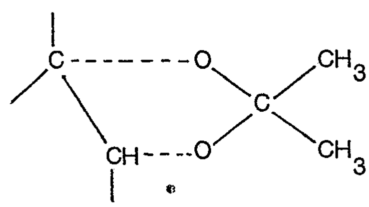 ,

ainsi que les sels qu'ils forment avec des bases acceptables du point de vue physiologique.

5. Sulfopropionates en 21 de corticoïdes qui répondent à la formule générale I selon la revendication 1 et qui sont caractérisés en ce que .... représente une liaison simple ou une liaison double, X représente un atome d'hydrogène ou un radical méthyle, Y représente un atome d'hydrogène, Z représente un atome d'hydrogène ayant la configuration $\alpha$ et un groupe hydroxy ayant la configuration $\beta$, et

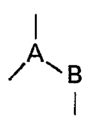 représente le groupement 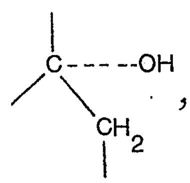 ,

ainsi que les sels qu'ils forment avec des bases acceptables du point de vue physiologique.

6. Sulfopropionates en 21 de corticoïdes qui répondent à la formule générale I selon la revendication 1 et qui sont caractérisés en ce que —A—B— représente le groupement

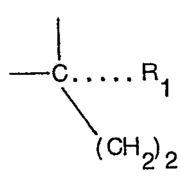 .

7. Sels de lithium, de sodium, de potassium, de calcium et d'ammonium de sulfopropionates en 21 de corticoïdes de formule I ou II selon l'une quelconque des revendications 1 à 6.

8. Procédé de préparation de sulfopropionates en 21 de corticoïdes répondant à la formule générale I selon la revendication 1 procédé caractérisé en ce qu'on fait réagir un corticoïde répondant à la formule générale I:

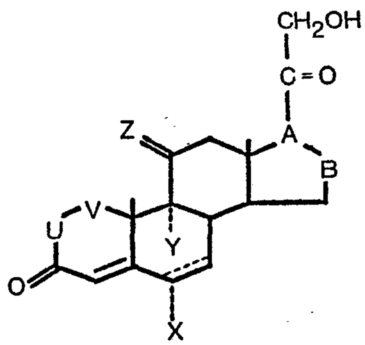

(II),

dans laquelle ...., —U—V—, X, Y, Z et —B—A— ont les significations données à la revendication 1, avec l'anhydride sulfo-3 propionique, corps qui répond à la formule III

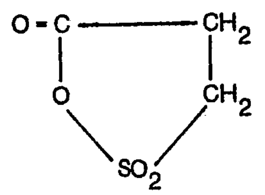

(III),

et on transforme éventuellement les composés obtenus, qui sont des sulfopropionates en 21 de corticoïdes, en leurs sels avec des bases acceptables du point de vue physiologique.

9. Compositions pharmaceutiques caractérisées en ce qu'elles contiennent un sel d'un sulfopropionate en 21 de corticoïde selon l'une quelconque des revendications 1 à 8.

10. Fluoro-6α hydroxy-11β méthyl-16α (sulfo-3 propionyloxy-21 prégnadiène-1,4 dione-3,20 et ses sels de sodium, de potassium, de lithium et d'ammonium.

11. Difluoro-6α,9α hydroxy-11β méthyl-16α (sulfo-3 propionyloxy)-21 prégnadiène-1,4 dione-3,20 et ses sels de sodium, de potassium, de lithium, de calcium et d'ammonium.

12. Dihydroxy-11β,17α (sulfo-3 propionyloxy)-21 prégnadiène-1,4 dione-3,20 et son sel sodique.

13. Dihydroxy-11β,17α méthyl-6α (sulfo-3 propionyloxy)-21 prégnadiène 1,4 dione-3,20 et son sel sodique.

14. Fluoro-9α hydroxy-11β isopropylidène-dioxy- 16α,17α (sulfo-3 propionyloxy)-21 prégnadiène-1,4 dione-3,20 et son sel sodique.

15. Fluoro-9α dihydroxy-11β,17α méthyl-16α (sulfo-3 propionyloxy-21 prégnadiène-1,4 dione-3,20 et son sel sodique.

16. Chloro-9α fluoro-6α hydroxy-11β méthyl-16α (sulfo-3 propionyloxy-21 prégnadiène-1,4 dione-3,20 et son sel sodique.

17. Fluoro-9α hydroxy-11β méthyl-16β (sulfo-3 propionyloxy)-21 prégnadiène-1,4 dione-3,20 et son sel sodique.

18. Fluoro-9α dihydroxy-11β,17α méthyl-16β (sulfo-3 propionyloxy)-21 prégnadiène-1,4 dione-3,20 et son sel sodique.

19. Dihydroxy-11β,17α (sulfo-3 propionyloxy)-21 prégnène-4 dione-3,20 et son sel sodique.

20. Fluoro-9α hydroxy-11β méthyl-16β valéryloxy-17α (sulfo-3 propionyloxy-21 prégnadiène-1,4 dione-3,20 son sel sodique et son sel potassique.

21. Fluoro-9α dihydroxy-11β,17α (sulfo-3 propionyloxy-21 prégnène-4 dione-3,20 et son sel sodique.

22. Butyryloxy-17α hydroxy-11β (sulfo-3 propionyloxy)-21 prégnène-4 dione-3,20 et son sel sodique.

23. Fluoro-6α dihydroxy-11β,17α (sulfo-3 propionyloxy-21 prégnadiène-1,4 dione-3,20 et son sel sodique.

24. Dihydroxy-11β,17aα (sulfo-3 propionyloxy)-21 D-homo-prégnadiène-1,4 dione-3,20 et son sel sodique.

25. Fluoro-9α dihydroxy-11β,17aα (sulfo-3 propionyloxy)-21 D-homo-prégnadiène-1,4 dione-3,20 et son sel sodique.

26. Chloro-6 dihydroxy-11β,17α (sulfo-3 propionyloxy)21 prégnatriène-1,4,6 dione-3,20 et son sel sodique.

27. Chloro-6 dihydroxy-11β,17α méthylène-1α, 2α (sulfo-3 propionyloxy)-21 prégnadiène-4,6 dione-3,20 et son sel sodique.

## Claims

1. Corticoid-21-sulphopropionates of the general formula I

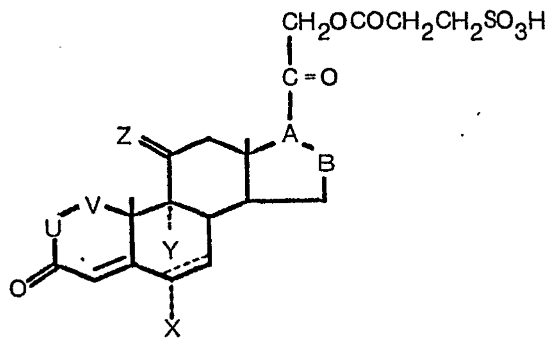

(I)

in which ..... represents a single bond or a double bond
X represents a hydrogen atom, a chlorine atom, a fluorine atom or a methyl group,
Y represents a hydrogen atom, a fluorine atom or a chlorine atom,
Z represents an oxo group or a hydrogen atom in the $\alpha$-position and a hydroxy group in the $\beta$-position,
—U—V— represents the groupings $-CH_2-CH_2-$,

CH=CH or 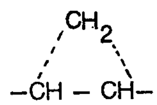 and 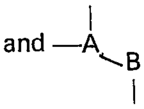 represents the groupings

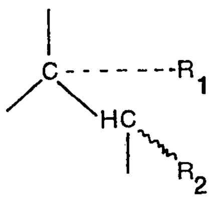 , 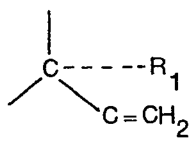 , 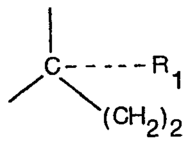 or 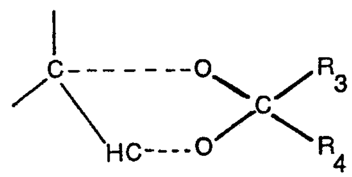

$R_1$ representing a hydrogen atom, a hydroxy group or an acyloxy group containing from 1 to 8 carbon atoms,
$R_2$ representing a hydrogen atom or a methyl group and
$R_3$ and $R_4$ representing an alkyl group containing from 1 to 4 carbon atoms
and salts thereof with physiologically tolerable bases.

2. Corticoid-21-sulphopropionates of the general formula I according to claim 1, characterised in that ..... represents a double bond, X represents a fluorine atom, Y represents a hydrogen atom, a fluorine atom or a chlorine atom, Z represents a hydrogen atom in the $\alpha$-position and a hydroxy group in the $\beta$-position and

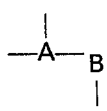 represents the grouping 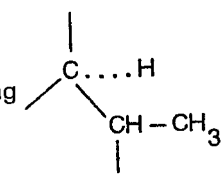

and salts thereof with physiologically tolerable bases.

3. Corticoid-21-sulphopropionates of the general formula I according to claim 1, characterised in that .... represents a double bond, X represents a hydrogen atom, Y represents a fluorine atom or a chlorine atom, Z represents a hydrogen atom in the $\alpha$-position and a hydroxy group in the $\beta$-position and

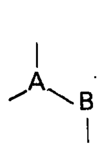 represents the grouping 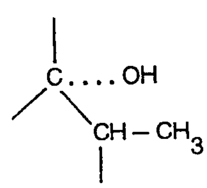

and salts thereof with physiologically tolerable bases.

4. Corticoid-21-sulphopropionates of the general formula I according to claim 1, characterised in that .... represents a double bond, X represents a hydrogen atom or a fluorine atom, Y represents a hydrogen atom or a fluorine atom, Z represents a hydrogen atom in the $\alpha$-position and a hydroxy group in the $\beta$-position and

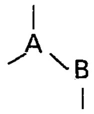 represents the grouping 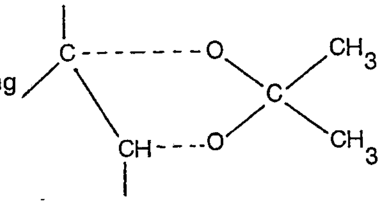

and salts thereof with physiologically tolerable bases.

5. Corticoid-21-sulphopropionates of the general formula I according to claim 1, characterised in that .... represents a single bond or a double bond, X represents a hydrogen atom or a methyl group, Y represents a hydrogen atom, Z represents a hydrogen atom in the $\alpha$-position and a hydroxy group in the $\beta$-position and

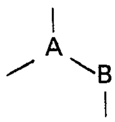 represents the grouping 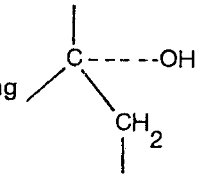

and salts thereof with physiologically tolerable bases.

6. Corticoid-21-sulphopropionates of the general formula I according to claim 1, characterised in that —A—B— represents the grouping

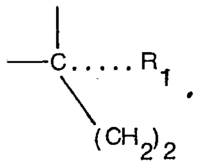

7. Lithium, sodium, potassium, calcium and ammonium salts of corticoid-21-sulphopropionates of the general formulae I according to claims 1 to 6.

8. Process for the manufacture of corticoid-21-sulphopropionates of the general formula I according to patent claim 1, characterised in that a corticoid of the general formula II

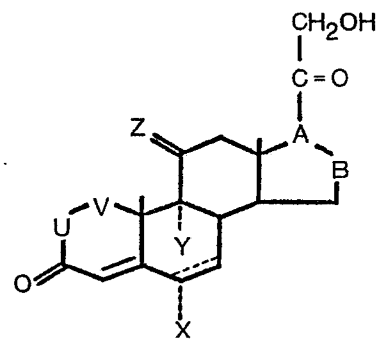

(II),

in which ....., —U—V—, X,Y,Z and —B—A— have the meanings given in claim 2, is reacted with 3-sulphopropionic acid anhydride of the formula III

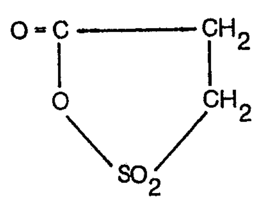

(III),

and the resulting corticoid-21-sulphopropionates are optionally converted with physiologically tolerable bases into their salts.

9. Pharmaceutical preparations, characterised in that they contain a salt of a corticoid-21-sulphopropionate according to claims 1 to 8.

10. 6α-Fluoro-11β-hydroxy-16α-methyl-21-(3-sulphopropionyloxy)-1,4-pregnadien-3,20-dione and the sodium, potassium, lithium and ammonium salts thereof.

11. 6α,9α-Difluoro-11β-hydroxy-16α-methyl-21-(3-sulphopropionyloxy)-1,4-pregnadien-3,20-dione and the sodium, potassium, lithium, calcium and ammonium salts thereof.

12. 11β,17α-Dihydroxy-21-(3-sulphopropionyloxy)-1,4-pregnadien-3,20-dione and the sodium salt thereof.

13. 11β,17α-Dihydroxy-6α-methyl-21-(3-sulphopropionyloxy)-1,4-pregnadien-3,20-dione and the sodium salt thereof.

14. 9α-Fluoro-11β-hydroxy-16α,17α-isopropylidenedioxy-21-(3-sulphopropionyloxy)-1,4-pregnadien-3,20-dione and the sodium salt thereof.

15. 9α-Fluoro-11β,17α-dihydroxy-16α-methyl-21-(3-sulphopropionyloxy)-1,4-pregnadien-3,20-dione and the sodium salt thereof.

16. 9α-Chloro-6α-fluoro-11β-hydroxy-16α-methyl-21-(3-sulphopropionyloxy)-1,4-pregnadien-3,20-dione and the sodium salt thereof.

17. 9α-Fluoro-11β-hydroxy-16α-methyl-21-(3-sulphopropionyloxy)-1,4-pregnadien-3,20-dione and the sodium salt thereof.

18. 9α-Fluoro-11β,17α-dihydroxy-16β-methyl-21-(3-sulphopropionyloxy)-1,4-pregnadien-3,20-dione and the sodium salt thereof.

19. 11β,17α-Dihydroxy-21-(3-sulphopropionyloxy)-4-pregnen-3,20-dione and the sodium salt thereof.

20. 9α-Fluoro-11β-hydroxy-16β-methyl-17α-valeryloxy-21-(3-sulphopropionyloxy)-1,4-pregnadien-3,20-dione and the sodium and potassium salts thereof.

21. 9α-Fluoro-11β,17α-dihydroxy-21-(3-sulphopropionyloxy)-4-pregnen-3,20-dione and the sodium salt thereof.

22. 17α-Butyryloxy-11β-hydroxy-21-(3-sulphopropionyloxy)-4-pregnen-3,20-dione and the sodium salt thereof.

23. 6α-Fluoro-11β,17α-dihydroxy-21-(3-sulphopropionyloxy)-1,4-pregnadien-3,20-dione and the sodium salt thereof.

24. 11β,17aα-Dihydroxy-21-(3-sulphopropionyloxy)-D-homo-1,4-pregnadien-3,20-dione and the sodium salt thereof.

25. 9α-Fluoro-11β,17aα-dihydroxy-21-(3-sulphopropionyloxy)-D-homo-1,4-pregnadien-3,20-dione and the sodium salt thereof.

26. 6-Chloro-11β,17α-dihydroxy-21-(3-sulphopropionyloxy)-1,4,6-pregnatrien-3,20-dione and the sodium salt thereof.

27. 6-Chloro-11β,17α-dihydroxy-1α,2α-methylene-21-(3-sulphopropionyloxy)-4,6-pregnadien-3,20-dione and the sodium salt thereof.